# EUROPEAN PATENT SPECIFICATION

(11) **EP 3 984 487 B1**
(45) Date of publication and mention of the grant of the patent: **14.05.2025**
(21) Application number: 20201992.3
(22) Date of filing: 15.10.2020
(51) Int. Cl.: A61B 34/20, A61B 34/10, A61B 34/00, A61B 90/00, A61B 17/00

(54) **TECHNIQUE FOR VISUALIZATION OF A SPHERICALLY SHAPED TIP OF A TRACKED INSTRUMENT RELATIVE TO A TRACKED OBJECT**
TECHNIK ZUR VISUALISIERUNG EINER SPHÄRISCH GEFORMTEN SPITZE EINES VERFOLGTEN INSTRUMENTS RELATIV ZU EINEM VERFOLGTEN OBJEKT
TECHNIQUE DE VISUALISATION D'UNE POINTE DE FORME SPHÉRIQUE D'UN INSTRUMENT SUIVI PAR RAPPORT À UN OBJET SUIVI

(43) Date of publication of application: 20.04.2022
(73) Proprietor: Stryker European Operations Limited, Carrigtwohill, Co. Cork T45 HX08 (IE)
(72) Inventor: GHANAM, Fadi, 79227 Schallstadt (DE)
(74) Representative: Röthinger, Rainer

(56) References cited:
- US-A1- 2012 109 152
- US-A1- 2019 142 527
- US-A1- 2020 046 454

## Description

### Technical Field

The present disclosure generally relates to instrument tracking, for example in the context of surgical navigation. In particular, a method, a computer program product, a device, a calibration system, and a surgical navigation system for enabling visualization a radius of a spherically shaped instrument tip are presented.

### Background

Surgical navigation systems are typically configured to track a surgical instrument operated by the surgeon relative to a patient or other object. Based on the tracked surgical instrument, the surgical navigation system provides visual or acoustic instructions to guide the surgeon when operating the surgical instrument.

A common procedure for surgical navigation is to track both the surgical instrument and the patient. The surgical navigation system can thus display image data of the patient (e.g., a computer tomography scan) relative to a visual representation of the surgical instrument or a part thereof. In such a visual representation, a tip of the surgical instrument is commonly represented in form of a point or icon.

Tissue manipulation such as cutting, milling or drilling is largely affected by a size of the instrument tip. The following two examples illustrated that visually representing the instrument tip in the form of a point can impair the accuracy of the guidance provided to the surgeon.

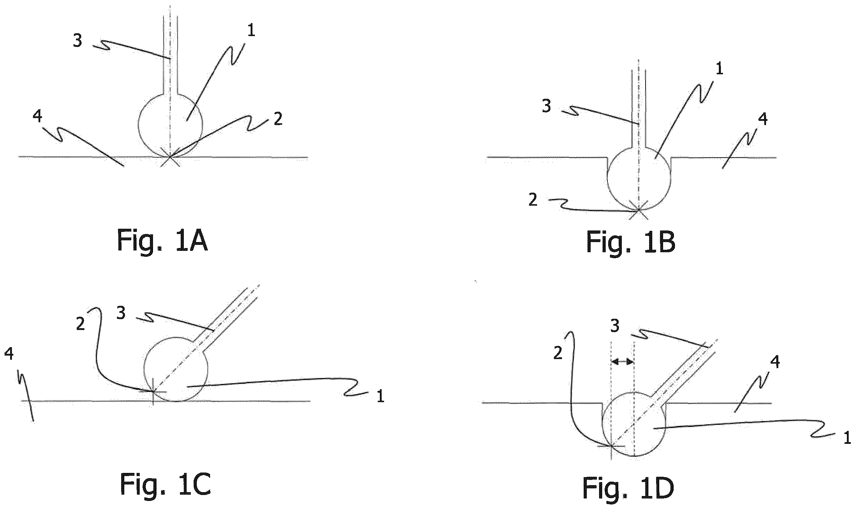

Fig. 1A shows a spherically shaped instrument tip 1 (e.g., a burr) having a distal end point 2. An instrument axis 3 extending through the end point 2 is arranged perpendicularly to a surface of tissue 4 (e.g., bone). Fig. 1B shows an opening that has been cut by the instrument tip 1 into the tissue 4 in a direction parallel to the instrument axis 3. Since the instrument axis 3 extends parallel to the opening cut into the tissue 4, the end point 2 is positioned at a bottom of the opening.

When visually representing a calculated location of the end point 2 relative to the tissue 4 on a display, the location of the visualized end point 2 properly coincides with the bottom of the opening. As such, the surgeon is correctly informed about, for example, an extension and a depth of the opening.

Fig. 1C shows the same instrument tip 1 as shown in Fig. 1A, but with the instrument axis 3 oriented at a non-perpendicular angle relative to the surface of the tissue 4. Therefore, when cutting an opening into the tissue 4 perpendicular to the surface of the tissue 4, the instrument axis 3 does not extend parallel to the opening. Fig. 1D shows that cutting an opening into the tissue 4 in the scenario of Fig. 1C results in the path of the end point 2 to be arranged offset to the cutting path of the opening. When a surgical navigation system uses the end point 2 as reference for the cutting path, the calculated cutting path will by offset relative to the actual cutting path. As such, the extension of the cutting path cannot be properly visualized. Moreover, the calculated location of the end point 2 is no longer indicative of a depth of the opening.

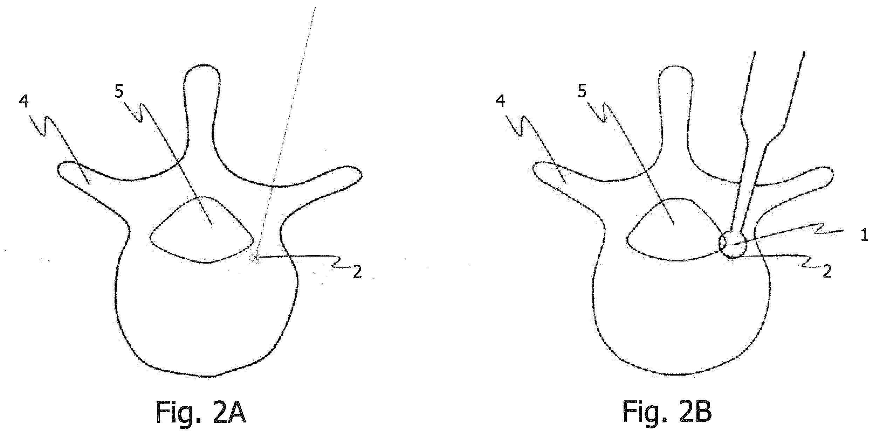

Fig. 2A illustrates an exemplary visual guidance that may be provided to the surgeon on a display. The visual guidance includes displaying a visual representation of a spherically shaped instrument tip in the form of an end point 2 relative to image data of tissue 4. The image data comprises a non-target area 5 that must not be affected by the surgical instrument. The visual guidance shown in Fig. 2A visualizes the end point 2 outside the non-target area 5, seemingly indicating that the instrument is not cutting into the non-target area 5. Fig. 2B shows the actual tissue 4 with the actual instrument tip 1. Since the end point 2 shown in Fig. 2A does not account of the radius of the instrument tip 1, the visual guidance of Fig. 2A does not indicate that the instrument tip 1 is in fact cutting into the non-target area 5.

As described in the two scenarios above, a visual representation in form of a point does not take the form and the size of the instrument tip into account. Such an incomplete visualization can negatively affect the accuracy the guiding instructions provided by the surgical navigation system.

The surgical instrument described with reference to Figs. 1A to 1D and Figs. 2A and 2B has an instrument tip with a spherically shaped surface portion that defines a radius, which may be unknown or require verification. The radius of the instrument tip can differ due to the availability of instrument tips that may be selected by the surgeon depending on the prevailing surgical situation.

In order to provide the surgeon with accurate guiding information in the scenarios discussed above, and in other scenarios, the surgical navigation system requires precise knowledge of the radius of the spherically shaped surface portion. In some cases the instruments are provided with labels that indicate the radius of their tips.

However, relying on the surgeon to properly input the selected instrument or the associated tip radius via a keyboard or graphical user interface has turned out to be error-prone and tedious during on-going surgery. Moreover, the use of labels does not take into account a radius change caused by wear or other effects. As a consequence, relying on the surgeon to properly identify the radius of a selected instrument tip is error prone, time-inefficient and potentially unsafe for the patient.

US 2019/0142527A1 discloses a method and system for computer assisted orthopedic feedback of at least one surgical object relative to a pre-operative plan.

The pre-operative plan includes a virtual object, which is the virtual representation of the surgical object, and a virtual structure, which is the virtual representation of the patient structure.

US 2020/0046454A1 discloses a registration and identification tool for an instrument, comprising a body, a marker member which is optically detectable and provided on the body, and a recess in the body which extends from an outer surface of the body into the inside of the body, thereby defining an extension direction of the recess, wherein the recess has a shape such that a lateral extension of the recess decreases in the direction from the outer surface of the body towards the inside of the body.

### Summary

There is a need for a technique that solves one or more of the aforementioned or other problems.

The invention is defined by the independent claims. Further embodiments are disclosed in the dependent claims.

According to one aspect, a method for enabling a visualization of an indication of a radius of a spherically shaped surface portion of a tip of an instrument relative to image data of an object in a virtual space is provided. The spherically shaped surface portion has a centre point. A calibration device is provided that comprises a calibration structure defining an opening angle and that is configured to cooperate with the spherically shaped surface portion so as to guide a tilting movement of the instrument tip around the centre point. The calibration device comprises a first tracker trackable by a tracking system and arranged in a predetermined relationship relative to the calibration structure. The instrument comprises a second tracker and the object comprises a third tracker trackable by the tracking system. The method is performed by a computer system and comprises determining a first pose of the first tracker. The method further comprises determining positional data of the second tracker for different tilting positions of the instrument while the spherically shaped surface portion is in abutment with the calibration structure. The method also comprises determining the radius of the spherically shaped surface portion and a position of the centre point relative to the second tracker based on the first pose, the positional data, the predetermined relationship, and the opening angle. The method comprises tracking the third tracker to track a second pose of the image data of the object in the virtual space. The method comprises tracking the second tracker to track a position of the centre point in the virtual space based on the position of the centre point relative to the second tracker. Further still, the method comprises generating first display instructions for displaying in the virtual space, based on the second pose of the image data of the object in the virtual space, the position of the centre point in the virtual space and the radius, a visual representation indicative of the radius relative to the image data of the object.

As understood herein, the display instructions are configured to control a display such that certain information is visually output to a user. As such, the display instructions will typically take the form of analog or digital signals.

The visual representation indicative of the radius may have been generated (e.g., scaled) such that the display properly reflects the actual location of the spherically shaped surface portion relative to the actual location of the object (e.g., relative to a surface portion of the object). As such, the image data of the object and the radius indication may be converted to a common scale for visualization.

The calibration structure may be configured to centre the centre point onto a central axis of the calibration structure. The object may be a surgical object, such as a second instrument or a patient. The image data of the object may be captured using a conventional camera (e.g., a stereo camera), computer tomography, magnetic resonance imaging or any other medical imaging procedure.

The tracking system may be configured to apply one or more of an optical tracking technique, an electromagnetic tracking technique and an ultrasound tracking technique. The optical tracking technique may rely on one or both of infrared and visible light. The initial calibration steps may be performed using an optical tracking technique and the later tracking steps may be performed using an electromagnetic tracking technique, or vice versa.

The method may comprise determining the radius by multiplying the sinus of half of the opening angle with a distance between the position of the centre point and a real or imaginary point relative to which the opening angle is defined. The distance between the position of the centre point and the real or imaginary point may be determined based on the first pose, the predetermined relationship, the positional data and the position of the centre point.

The calibration device may comprise a plurality of calibration structures, wherein the first tracker is arranged in a predetermined manner relative to each of the plurality of calibration structures. The method may further comprise determining the position and, optionally, the opening angle of the calibration structure in which the instrument tip is arranged by identifying the calibration structure based on the position of the centre point. The central axis of at least two calibration structures may be arranged parallel to each other. The central axis of at least two calibration structures may alternatively be arranged non-parallel to each other, such as at an angle of 90°.

The positional data may comprise at least two different third poses of the second tracker for the different tilting positions. Alternatively or additionally, the positional data may comprise at least four different positions of the second tracker for the different tilting positions.

The method may comprise receiving an expected radius value and validating whether the determined radius corresponds to the expected radius value. The validation may be performed based on a threshold difference between the expected radius value and the determined radius. The method may comprise receiving the expected radius value as user input (e.g., entered via a keyboard or graphical user interface). The method may further comprise generating display instructions for displaying a result of the validation.

The method may further comprise outputting, on a display, instructions to tilt the instrument for calibration purposes. The instructions may indicate when to start and/or to stop tilting the instrument.

The method may comprise generating second display instructions for displaying a non-target region in the image data for which contact with the instrument is to be avoided. The method may comprise outputting a warning when the instrument tip comes in contact with the non-target region and/or a threshold region around the non-target region. The warning may be at least one of an acoustic, optical, and haptic warning.

The method may comprise generating third display instructions for displaying a progress of object manipulation based on a path of the instrument tip. The progress of object manipulation may be based on a surface generated from a circle or sphere with the determined radius and a tracked movement of the centre point as a generatrix.

The method may comprise displaying, on a display, a visual representation (e.g., an icon) of at least a portion of the instrument relative to the image data of the object. The display may be part of the tracking system, a surgical navigation system or be a separate display that is communicatively coupled with the tracking system or the surgical navigation system.

The calibration structure may have a shape of a cone or a pyramid, or a portion thereof, defining the opening angle. The calibration structure may comprise plates or other rigid structures that form a (e.g., partial) cone or partial pyramid. The calibration structure may have a shape of a truncated cone or pyramid.

The visual representation may comprises an icon representative of the centre point and at least a part of a circle or a sphere with the determined radius of the spherically shaped surface portion around the icon. The icon may further be representative of the centre point with the determined radius of the spherically shaped surface portion around the centre point.

A pose of an instrument axis may be determined on the basis of tracking the second tracker while the instrument is rotated about the instrument axis. Alternatively, the pose of the instrument axis may be determined on the basis tracking the second tracker and evaluating a known predetermined spatial relationship between the second tracker and the instrument axis. The visual representation may be indicative of the pose of the instrument axis in the virtual space. The instrument axis may be represented in the virtual space as a line that extends towards the centre point of the spherically shaped surface portion.

According to a second aspect, a computer program product is provided, comprising instructions that, when executed on at least one processor, cause the at least one processor to carry out any of the methods described herein. The computer program product may be stored on a non-volatile memory, such as a hard disc drive, a flash memory, a read-only memory, or an optical disc.

According to a third aspect, a device for enabling visualization of an indication of a radius of a spherically shaped surface portion of a tip of an instrument relative to image data of an object in a virtual space is provided. The spherically shaped surface portion has a centre point. A calibration device is provided that comprises a calibration structure defining an opening angle and that is configured to cooperate with the spherically shaped surface portion so as to guide a tilting movement of the instrument tip around the centre point. The calibration device comprises a first tracker trackable by a tracking system and arranged in a predetermined relationship relative to the calibration structure and wherein the instrument comprises a second tracker and the object comprises a third tracker trackable by the tracking system. The device is configured to determine a first pose of the first tracker. The device is further configured to determine positional data of the second tracker for different tilting positions of the instrument while the spherically shaped surface portion is in abutment with the calibration structure. The device is configured to determine the radius of the spherically shaped surface portion and a position of the centre point relative to the second tracker based on the first pose, the positional data, the predetermined relationship, and the opening angle. The device is configured to track the third tracker to track a second pose of the image data of the object in the virtual space. The device is further configured to track the second tracker to track a position of the centre point in the virtual space based on the position of the centre relative to the second tracker. The device is configured to generate first display instructions for displaying in the virtual space, based on the second pose of the image data of the object in the virtual space, the position of the centre point in the virtual space and the radius, a visual representation indicative of the radius relative to the image data of the object.

The device may be configured to perform any of the method steps described herein. The device may be a computer system or a part of a computer system.

According to a fourth aspect, a calibration system is provided. The calibration system comprises the device for enabling a radius visualization as described herein. The calibration system may further comprise the calibration device with the first tracker coupled thereto and the instrument with the second tracker coupled thereto.

According to a fifth aspect a surgical navigation system is provided. The surgical navigation system comprises the calibration system as described herein. The surgical navigation system further comprises the tracking system, wherein the tracking system comprises or is communicatively coupled to the device for enabling a radius visualization.

### Brief Description of the Drawings

Further details, advantages and aspects of the present disclosure will become apparent from the following embodiments when taken in conjunction with the drawings, wherein:
- Fig. 1A: shows an instrument tip with an end point as virtual representation of an instrument tip;
- Fig. 1B: shows the instrument tip of Fig. 1A cutting an opening into tissue;
- Fig. 1C: shows the instrument tip of Fig. 1A with an instrument axis oriented in a non-perpendicular angle relative to the surface of tissue;
- Fig. 1D: shows the instrument tip in the orientation of Fig. 1C cutting an opening into the tissue;
- Fig. 2A: shows a visual guidance comprising image data of tissue and a visual representation of an instrument in form of an end point;
- Fig. 2B: shows the actual tissue with the corresponding actual location of the instrument tip in the scenario of Fig. 2A;
- Fig. 3: shows a surgical navigation system comprising a device embodiment;
- Fig. 4A-C: show enlarged views of spherically shaped instrument tips;
- Fig. 5A: shows a calibration device with a first tracker and a calibration structure defining an opening angle;
- Fig. 5B: shows a calibration device for electromagnetic tracking, wherein the first tracker comprises coils;
- Fig. 6A: shows the instrument tip arranged inside a calibration structure with the shape of a cone;
- Fig. 6B: shows a top view of the cone of the calibration structure shown in Fig. 6A and a circular contact region;
- Fig. 6C: shows a calibration structure with an inverted regular pyramid with three sides;
- Fig. 6D: shows a top view of the pyramid of the calibration structure shown in Fig. 6C with three contact regions;
- Fig. 7A: shows a calibration structure with an inverted regular pyramid with six sides;
- Fig. 7B: shows a top view of the pyramid of the calibration structure shown in Fig. 7A with six contact regions;
- Fig. 8: shows a flow diagram of a method for visualizing an indication of a radius of a spherically shaped surface portion;
- Fig. 9A: shows three different tilting positions of the surgical instrument while the spherically shaped surface portion is in abutment with the calibration structure;
- Fig. 9B: shows a side view of the spherically shaped surface portion in abutment with the calibration structure;
- Fig. 10: shows geometrical relationships between the tracking system, the surgical instrument, and the calibration structure;
- Fig. 11: shows an enlarged view of the spherically shaped surface portion in abutment with the calibration structure and geometric parameters relevant for determining the radius;
- Fig. 12: shows an enlarged view of two trackers being tracked by a tracking system;
- Figs. 13A-C: show virtual space examples each with a visual representation indicative of a radius being arranged relative to image data of the object;
- Fig. 14: shows a calibration device with a first calibration structure and a second calibration structure.

### Detailed Description

In the following description of exemplary embodiments, the same reference numerals are used to denote the same or similar components.

The embodiments described hereinafter are directed to enabling a visual indication of a spherical surface of an instrument tip relative to image data of an object. The instrument may be used in various technical fields such as in robotics, material science, or medical procedures. The following embodiments exemplarily relate to surgical navigation of a surgical instrument.

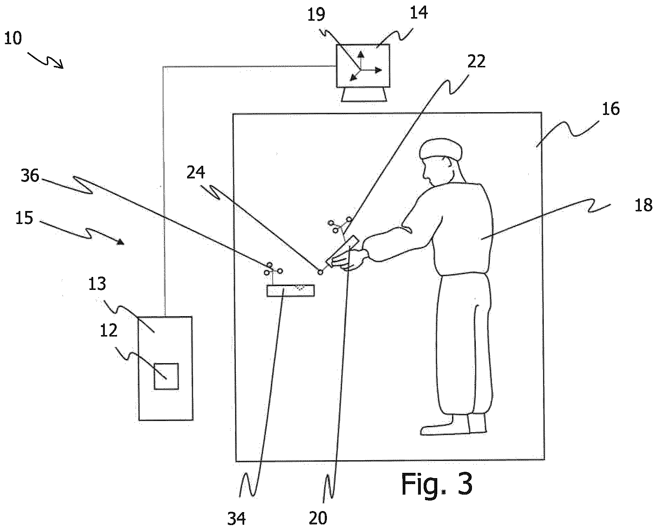

Fig. 3 shows an embodiment of a surgical navigation system 10 comprising a device 12 configured to determine a visualization for surgical navigation purposes. The device 12 shown in Fig. 3 may be comprised by a dedicated computer system 13. Alternatively, or in addition, the device 12 may by comprised by cloud computing resources.

The surgical navigation system 10 further comprises a tracking system 14. The tracking system 14 shown in Fig. 3 comprises a camera for optical tracking. Additionally or alternatively, the tracking system 14 comprises an electromagnetic field generator for electromagnetic tracking. The tracking system 14 is configured to gather tracking data within a surgical environment 16. The tracking data may take the form of images of the camera or electrical currents induced in an electromagnetic tracker (e.g., in one or more coils).

Fig. 3 also illustrates a user 18 (e.g., a surgeon or medical staff) moving a surgical instrument 20 relative to a calibration device 34 in the surgical environment 16. The associated calibration procedure targets at determining a tip radius of the surgical instrument 20. The device 12, the calibration device 34 and the surgical instrument 20 are part of a calibration system 15 comprised by the surgical navigation system 10.

As shown in Fig. 3, the calibration device 34 comprises a first tracker 36 and the surgical instrument 20 comprises a second tracker 22. The first and second trackers 36, 22 are both trackable by the tracking system 14. The first and second trackers 36, 22 exemplarily shown in Fig. 3 are passive trackers with reflective markers (e.g., reflective spheres). Alternatively, the first and second trackers 36, 22 may be active trackers with light emitting elements. Further alternatively, the first and second trackers 36, 22 may comprise coils for electromagnetic tracking. As a still further alternative, the surgical instrument 20 and the calibration device 34 as such may also constitute respective trackers 36, 22. For example, using a stereo camera of the tracking system 14, the tracking system 14 may simply track the known shapes of the one or both of the surgical instrument 20 and calibration device 34 for determining their positions or poses within the surgical environment 16.

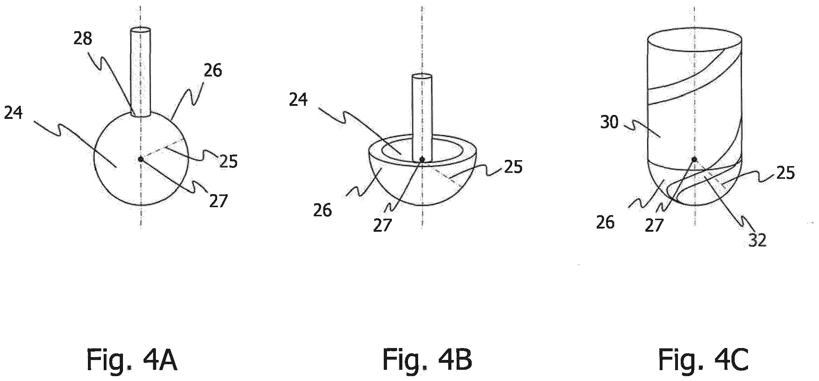

Fig. 4A shows an enlarged view of an exemplary instrument tip 24 of the surgical instrument 20. The instrument tip 24 has a radius 25 that is defined by a spherically shaped surface portion 26 around a centre point 27. The surface portion 26 may have openings or rake structures (e.g., in the form of cutting flutes) and may therefore not form one continuous surface. In some variants, the instrument tip 24 may be a burr.

The surface portion 26 of the instrument tip 24 shown in Fig. 4A forms almost a complete sphere with the exception of an attachment region 28 where the tool tip 24 is connected to a shaft of the surgical instrument 20. The shaft with the tool tip 24 may be removable from the surgical instrument 20, so that the user 18 may selectively use the surgical instrument 20 with different tool tips 24 having different radii.

Alternatively to the example illustrated in Fig. 4A, the spherically shaped surface portion 26 may form a smaller portion of a sphere. Fig. 4B shows an instrument tip 24 with a surface portion 26 that forms a half sphere. The surface portion 26 may form any other portion of a sphere such as a third, or a quarter of a sphere. Fig. 4C shows an instrument tip 24 in the form of a drill bit with an essentially spherically shaped surface portion 26 and an essentially cylindrical surface portion 30. The instrument tip 24 comprises one or more grooves 32 that interrupt a continuous surface of the spherically shaped surface portion 26.

In the examples illustrated in Figs. 4A to 4C, the spherically shaped surface portion 26 defines a virtual sphere with the radius 25 and the centre point 27. Points on the surface portion 26 are arranged with a distance of the radius 25 away from the common centre point 27. Prior to actual surgery, the radius 25 of the spherically shaped surface portion 26 will need to be determined by the user 18 using the calibration device 34.

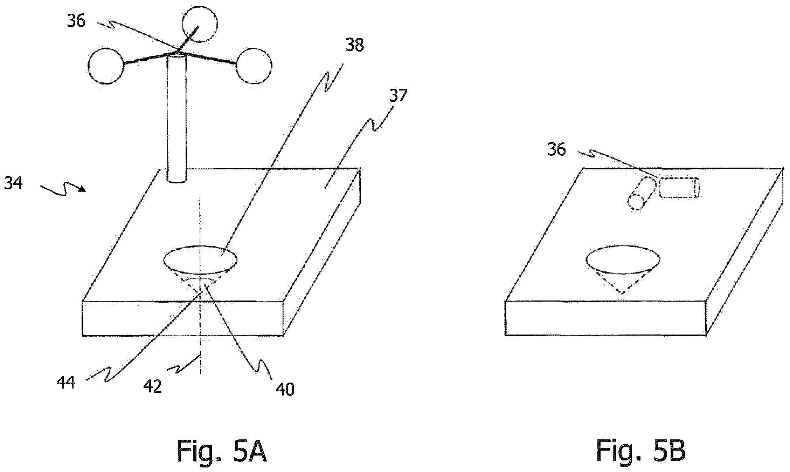

Fig. 5A shows an example of the calibration device 34 with the first tracker 36 and a calibration structure 38 defining an opening angle 40. The first tracker 36 needs to be trackable by the tracking system 14 of the surgical navigation system 10. Therefore, in the above example of an optical navigation system 10, the first tracker 36 is an optical tracker as shown in Fig. 5A with three (or more) reflective spheres. In case the surgical navigation system 10 employs electromagnetic tracking, the first tracker 36 may comprise one, two or more coils as shown in Fig. 5B.

The calibration structure 38 shown in Fig. 5A is formed as a cone-shaped surface opening in a plate-shaped body 37. The resulting cone 38 defines a central axis 42 through an apex 44 of the cone 38. The cone 38 has an opening angle 40 that is defined as twice the angle between the central axis 42 and any surface line of the cone 38 lying in the same plane as the central axis 42. The opening angle 40 may also be defined as the largest angle between two surface lines of the cone 38 lying in the same plane as the central axis 42. The opening angle 40 may be between 30° and 150°, such as between 80° and 100°. The opening angle may for example, be selected to be 45°, 90° or 120°.

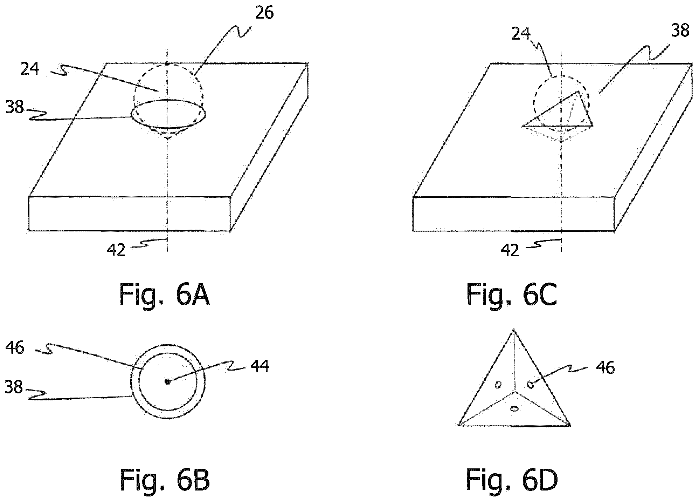

Fig. 6A shows the instrument tip 24 arranged inside the cone-shaped calibration structure 38 of Fig. 5A. As shown in Fig. 6B, the cone 38 is a circular cone, which means a plane perpendicular to the central axis 42 intersects the cone not in an oval but a circle. Consequently, the spherically shaped surface portion 26 abuts against the cone 38 in a circular contact region. Fig. 6B shows a top view of the cone 38 shown in Fig. 6A and the circular contact region 46. In the top view, a centre of the circular contact region 46 aligns with the apex 44 and therefore with the central axis 42. Due to the rotational symmetry of the contact region 46 and the spherically shaped surface portion 26, the centre point 27 of the spherically shaped surface portion 26 is centred by the cone 38 onto the central axis 42. The centre point 27 is arranged on the central axis 42 independently of the radius 25 of the surface portion 26 (as long as the surface portion 26 fits into the cone 38). However, the radius 25 correlates with a distance of the centre point 27 relative to the apex 44 of the cone 38. A smaller radius 25 of the surface portion 26 results in a shorter distance between the centre point 27 and the apex 44 compared to a larger radius 25 of the surface portion 26 that results in a larger distance between the centre point 27 and the apex 44.

The centring function of the calibration structure 38 does not necessarily require a cone shaped opening. Alternatively, the calibration structure 38 can have an opening with the shape of a regular pyramid, which means that a plane perpendicular to a central axis of the pyramid intersects the pyramid in a regular polygon (with equal side lengths and inner angles). As an example, the pyramid can have three, four, five, or more sides. Fig. 6C shows a pyramidal calibration structure 38 with an inverted regular pyramid with three sides. Fig. 6D shows a top view of the pyramid of the calibration structure 38 and three contact regions 46.

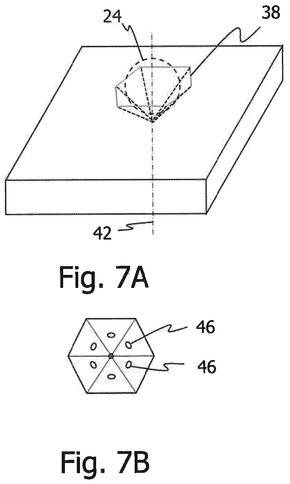

Fig. 7A shows another example of a pyramidal calibration structure 38 with an inverted regular pyramid with six sides. Fig. 7B shows a top view of the pyramid of the calibration structure 38 and six contact regions 46. The contact regions of the pyramid are positioned along a circle with a centre point that is arranged on a central axis 42 of the pyramid. For a pyramid, the opening angle is defined as twice the angle between a pyramid side and the central axis 42. In case of a pyramid with an even amount of sides, the opening angle is also the angle between two opposite pyramid sides.

A calibration structure 38 in the shape of a cone or a pyramid with at least three sides, such as described above, results in a circular contact region or at least three contact regions that confine the centre point 27 of the spherically shaped surface portion 26 onto a single point.

The various calibration structures 38 described herein are capable of receiving the instrument tip 24 and are configured to cooperate with the spherically shaped surface portion 26 so as to guide a tilting movement of the instrument tip 24 around the centre point 27 of the spherically shaped surface portion 26. In the process of guiding the tilting movement, the spherically shaped surface portion 26 slides along the calibration structure 38. Therefore, the calibration structure 38 preferably comprises a hard material with a Young's modulus larger than 150 GPa such as ceramics or metal (e.g., stainless steel).

As can be seen if Figs. 3 and 5A, the tracker of the calibration device 36 is arranged in a predetermined relationship relative to the calibration structure 38. The predetermined relationship may be defined by a pose (i.e., orientation and position) of the tracker 36 relative to the apex 44 of the calibration structure. The predetermined relationship may, for example, be defined as a matrix transformation in a coordinate system 19 (see Fig. 3) of the tracking system 14 that maps a pose of the tracker 36 onto the apex 44 (or vice versa). Alternatively or additionally, the surgical navigation system 10 may have access to geometrical properties (e.g., a digital model) of the calibration device 34.

As will be described below, the calibration device 34 will be used for determining the radius 25 of the spherically shaped surface portion 26 in an initial calibration procedure. Based on the radius 25 thus determined, display instructions can then be generated during a later surgical procedure to cause a display to output a visual representation indicative of the determined radius 25 relative to image data of a patient.

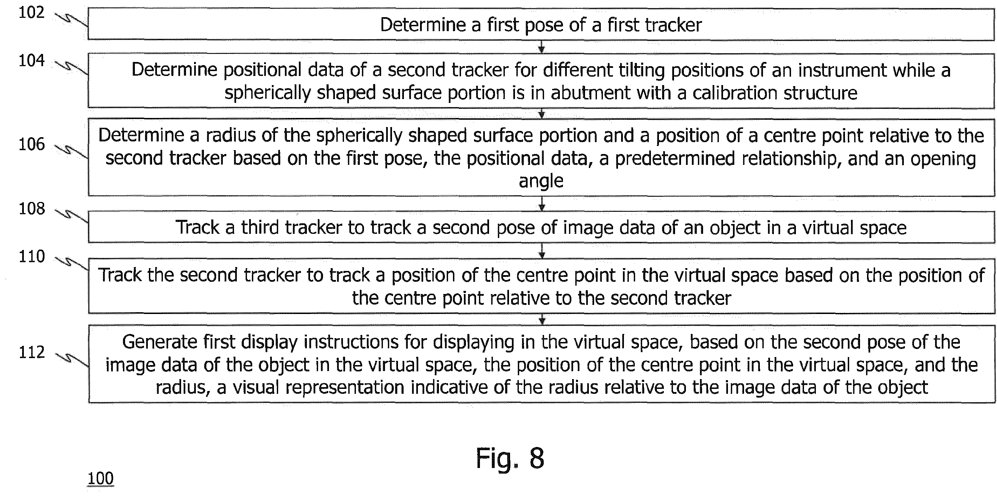

Fig. 8 shows a flow diagram of an embodiment of method 100 for enabling a visual indication of a radius 25 of the spherically shaped surface portion 26 of the tip 24 of the surgical instrument 20 using the calibration device 36.

The method comprises determining, in step 102, a pose of the tracker 36. The tracker first comprises a position and orientation of the tracker 36 and may be determined in the coordinate system 19 of the tracking system 14. Any technique known in the prior art may used for determining the pose of the tracker 36. For optical tracking, a common approach is to capture one or more images of the reflective markers of the tracker 36 and determine the pose based on a known geometric arrangement of the markers relative to each other (Fig. 5A). For electromagnetic tracking, a common approach is for a field generator of the surgical navigation system 10 to generate an electric field that induces currents in coils constituting the tracker 36 (Fig. 5B). Based on a measurement of the induced currents, the pose of the tracker 36 may be determined.

The method further comprises determining, in step 104, positional data of the instrument tracker 22 (see Fig. 3) for different tilting positions of the surgical instrument 20 while the spherically shaped surface portion 26 remains in abutment with the calibration structure 38. The positional data may comprise at least two different poses of the instrument tracker 22 for the different tilting positions or at least four different positions of the instrument tracker 22 for the different tilting positions.

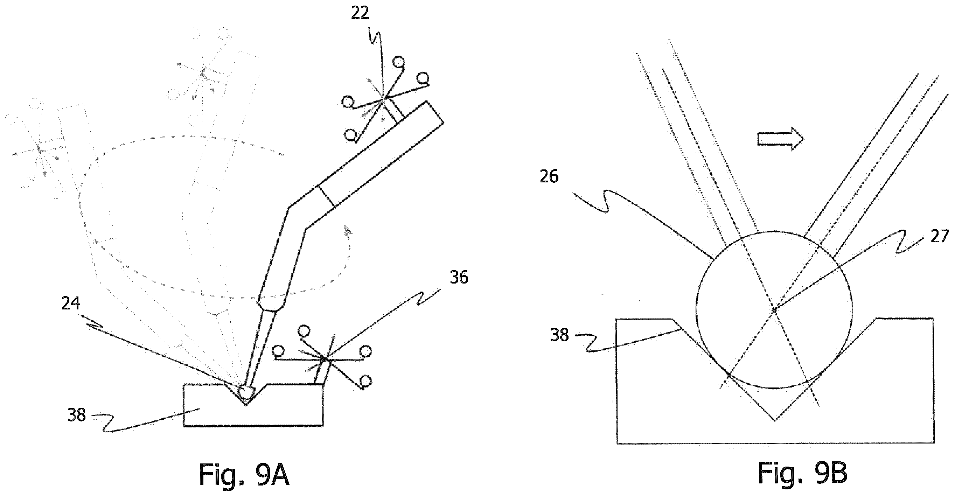

Fig. 9A shows examples of three different tilting positions of the surgical instrument 20 while the spherically shaped surface portion 26 remains in abutment with the calibration structure 38. The different tilting positions of the surgical instrument 20 cause the instrument tracker 22 to move along a sphere around the centre point 27. The position and orientation of the tracker 36 coupled to the calibration device 34 is, on the other hand, not affected by the different tilting positions of the surgical instrument 20.

The method optionally comprises generating user instructions for displaying on a display of the surgical navigation system 10 (or of another computing device communicatively connected with the surgical navigation system 10) instructions to tilt the surgical instrument 20 for calibration purposes. Such user instructions will initially request the user 18 to start moving the surgical instrument 20 for acquisition of pose or position data and will inform him or her once sufficient data for radius calculation have been acquired.

The method further comprises determining, in step 106, the radius 25 of the spherically shaped surface portion 26 and a position of the centre point 27 relative to the instrument tracker 22. The determination in step 106 is based on the pose of the tracker 36, the positional data acquired for the instrument tracker 22, the predetermined relationship between the tracker 36 and the calibration structure 38, and the opening angle 40 of the calibration structure 38..

According to geometrical principles, the radius 25 can be determined based on the position of the centre point 27 of the instrument tip 24. The positional data acquired for the instrument tracker 22 can be used in this regard to determine a position of the centre point 27 of the spherically shaped surface portion 26. Depending on whether the positional data comprises poses of the instrument tracker 22 or only positions of the instrument tracker 22, two different approaches can be performed in order to determine the position of the centre point 27.

In the first approach, the positional data comprises at least two different poses of the instrument tracker 22 for the different tilting positions. Fig. 9B shows a side view of the spherically shaped surface portion 26 in abutment with the calibration structure 38. While the instrument tip 24 remains in abutment with calibration structure 38, the calibration structure 38 guides tilting of the instrument tip 24 (and, thus, of the surgical instrument 20 and the instrument tracker 22) around the centre point 27. Therefore, two different poses of the instrument tracker 22 for two different tilting positions differ (at least partly) in a rotation around the centre point 27. The position of the centre point 27 may be determined based on a transformation comprising a rotational transformation that maps one of the two poses onto the other pose. The position of centre point 27 is a position of unity (i.e., the only position unaltered by the transformation) of the rotation transformation.

Another way to determine the position of the centre point 27 based on the two poses is to determine for each marker of the instrument tracker 22 a linear vector that translates the marker between the two poses. For each translation vector a halfway point is further determined that is arranged in the centre between the positions of the marker for each of the poses. Due to the poses being arranged on a sphere, a connection vector from the halfway point to the (yet unknown) centre point 27 is arranged orthogonally to the direction of the translation vector. Therefore, a scalar product between the connection vector and the translation vector is zero. This allows defining, for each reflective marker of the instrument tracker 22, an equation that includes the position of the centre point 27 (in form of the connection vector). These equations can be solved for the position of the centre point 27.

It should be noted that the calculations described above require at least two poses of the instrument tracker 22. To increase the overall precisions, more than two poses may be captured, and determining the position of the centre point 27 may be performed with the more than two poses. A final position of the centre point 27 may be calculated from an average of multiple positions of the centre point 27 determined for the more than two poses of the instrument tracker 22. Determining the centre point 27 on the basis of more than two poses generally decreases a statistical error of the calculation.

In the second approach, the positional data comprises at least four different positions of the instrument tracker 22 for the different tilting positions. When the surgical instrument 20 is tilted around the centre point 27, the position of a centre of the instrument tracker 22 is moved on a sphere around the centre point 27. A sphere can be defined by, and reconstructed from, four points of the sphere (not arranged in a common plane). Therefore, the four positions of the instrument tracker 22 contain enough information for determining the sphere and, consequently, the position of its centre point 27. One way to determine the centre point 27 is to set up equations that define a distance (e.g., via Pythagorean equations) between the four tracker positions relative to the unknown point centre 27 and equate the distance to an unknown radius. The four equations include four unknown variables (i.e., three coordinates of the position of the centre point 27 and the radius) and therefore form a determined system that is mathematically solvable. It should be noted that the radius of the sphere in these equations is not the radius of the spherically shaped surface portion 26, but the distance between the centre point 27 and the centre of the instrument tracker 22. In order to reduce the statistical error of the calculation, the position of the centre point 27 may of course be determined based on more than four positions of the instrument tracker 22.

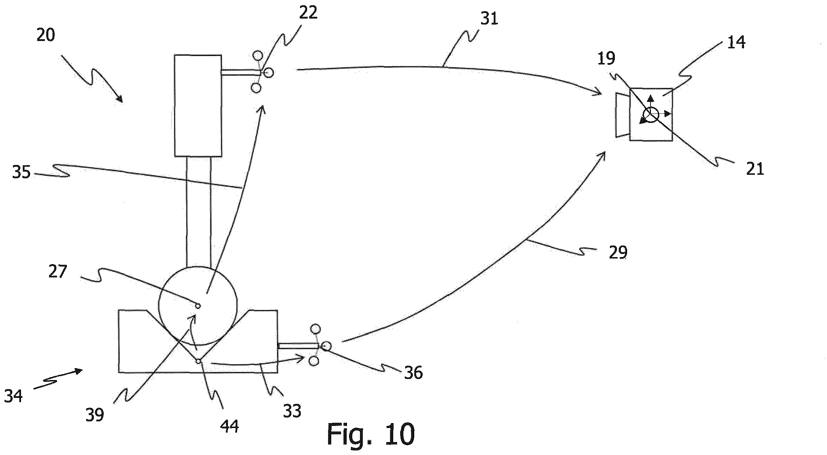

Based on the pose of the tracker 36 of the calibration device 36 and the predetermined relationship between that tracker 36 and the calibration structure 38, a position of the apex 44 of the calibration structure 38 may be determined. Fig. 10 shows geometrical relationships between the tracking system 14, the surgical instrument 20, and the calibration structure 36. The geometrical relationships are in the following described as transformations that map one point in space onto another point in space. The transformations may, for example, be defined as mathematical transformations such as a matrix. It should be noted that any transformation as described herein may alternatively be defined as its inverse transformation.

The tracking system 14 in configure to determine a pose of the instrument tracker 22 in form of a first transformation 31 and a pose of the tracker 36 of the calibration device 36 in form of a second transformation 29. The first and second transformations 31, 29 may be defined as transformations from centres of each of the two trackers 36, 22 to a centre point 21 of the coordinate system 19 of the tracking system 14. The centre point 21 may be positioned in an image plane of the camera of the tracking system 14.

The relationship between the tracker 36 of the calibration device 36 and the calibration structure 38 shown in Fig. 10 may be a geometric relationship as defined by a third transformation 33 that maps the apex 44 onto the centre of that tracker 36. A fourth transformation 35 can be defined based on the determined position of the centre point 27 and a pose determined for the instrument tracker 22.

Further, a fifth transformation 39 can be defined that maps the centre point 27 onto the apex 44. As can be seen in Fig. 10, the first, second, third, fourth, and fifth transformations 31, 29, 33, 35, 39 jointly describe a closed circle of transformations. Consequently, the fifth transformation 39 can be determined by a sum of the first, second, third, and fourth transformations 31, 29, 33, 35. Based on the determined fifth transformation 39, an apex distance 56 between the position of the apex 44 and the position of the centre point 27 can be calculated. The apex distance 56 thus calculated can be used to determine the radius 25, as described below.

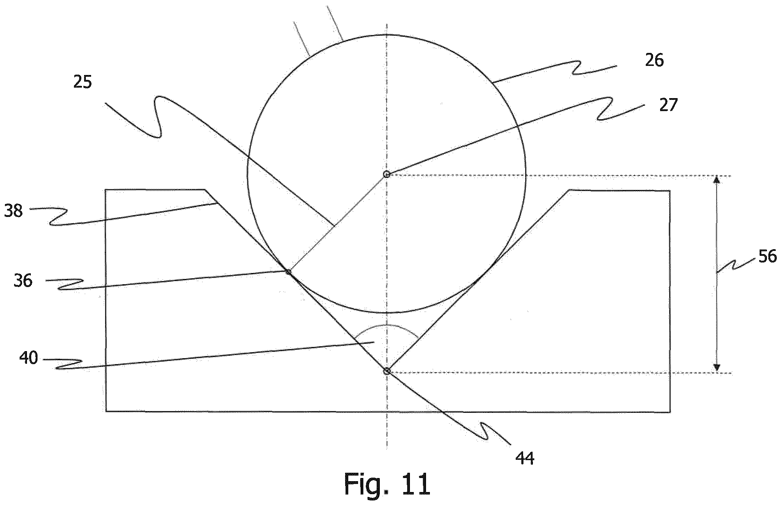

Fig. 11 shows an enlarged view of the spherically shaped surface portion 26 in abutment with the calibration structure 38 and geometric parameters relevant for determining the radius 25. As can be seen in Fig. 11, the radius 25 of the spherically shaped surface portion 26 can be calculated by multiplying the apex distance 56 with a sinus of half of the opening angle 40. It should be noted that in Fig. 11, the apex distance 56 can be defined as the distance between the centre point 27 and the apex 44, because the calibration structure 38 has an apex 44. In case the calibration structure 38 has a shape without an apex 44 such as a frustum (i.e., a truncated pyramid or cone), the apex distance 56 may generally be defined as a distance between the position of the centre point 27 and an imaginary point ("imaginary apex") relative to which the opening angle 40 is defined.

In a first step sequence of the method described above (i.e., in steps 102, 104 and 106 as illustrated in Fig. 8, "calibration procedure"), the radius 25 of the spherically shaped surface portion 26 has been determined. A second step sequence of the method described below (i.e., steps 108, 110 and 112 of Fig. 8, "surgical procedure") deals with generating display instructions for displaying a visual representation of the radius 25 in a virtual space. The second step sequence builds upon the first step sequence. Moreover, while one of the two sequences may be modified, the other sequence may remain unmodified.

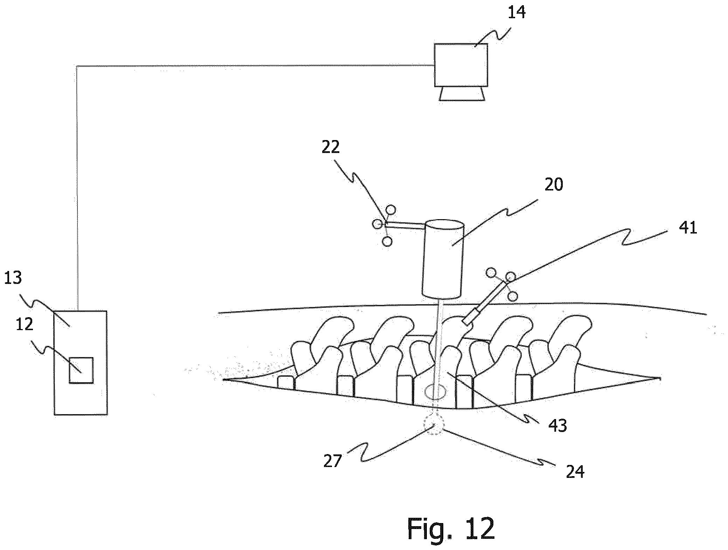

As such, the method further comprises tracking, in step 108 and during a surgical procedure, a further tracker to track a pose of image data of an object (e.g., a part of a patient anatomy) in the virtual space (e.g., defined by coordinates of the coordinate system 19 of the tracking system 14 or of a coordinate system of image data). Fig. 12 shows an enlarged view a surgical scenario with the instrument tracker 22 and a patient tracker 41 being tracked by the tracking system 14. The patient tracker 41 shown in Fig. 12 is coupled to an object 43 (in form of a vertebra) to be treated by the surgical instrument 20. As explained above, also the vertebra 43 itself may constitute the patient tracker 41.

The method also comprises tracking, in step 110, the instrument tracker 22 to track a position of the centre point 27 of the instrument tip 24 in the virtual space based on the position of the centre point 27 relative to the instrument tracker 22. For example, the position of the centre point 27 relative to the instrument tracker 22 may be described by the fourth transformation 35 (see Fig. 10) or otherwise. The position of the centre point 27 may then be tracked by tracking the instrument tracker 22 and applying the fourth transformation 35 onto the pose of a centre of the instrument tracker 22 thus determined.

As can be seen in Fig. 12, the instrument tip 24 is used to manipulate the vertebra 43 by burring or drilling a hole into the vertebra. However, from the outside, the user 18 has only a limited view of the manipulation occurring inside the vertebra 43. The method thus further comprises generating, in step 112, display instructions for displaying in the virtual space a visual representation indicative of the radius 25 relative to the image data of the vertebra 43. The display instructions are generated based on the pose of the image data of the vertebra 43 in the virtual space, the position of the centre point 27 in the virtual space, and the radius 25,.

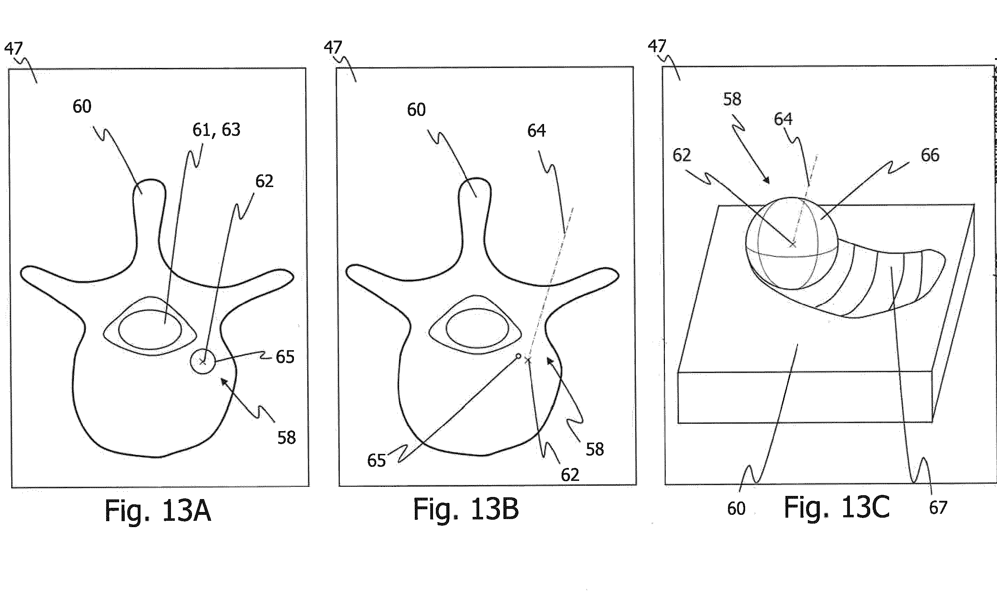

Fig. 13A shows a first example of a virtual space 47 with a visual representation 58 indicative of the radius 25 arranged relative to image data 60 of the vertebra 43 of Fig. 12. The image data 60 may, for example, comprise a point cloud or polygon mesh captured using computer tomography, magnetic resonance imaging, or any other medical imaging procedure. The image data 60 can be arranged in the virtual space 47 based on the pose of the image data, which was determined based on tracking the tracker 41. The visual representation 58 can be arranged in the virtual space 47 based on the tracked instrument tracker 22. Consequently, the image data 60 and the visual representation can be arranged relative to each other in the virtual space 47. The virtual space 47 may be defined, for example, by a coordinate system of the tracking system 14 or a coordinate system of the image data 60.

The exemplary visual representation 58 shown in Fig. 13A comprises the position of the centre point 27 and a circle 65 around the centre point 27 with the radius 25 as determined during the calibration procedure. Of course, in other examples, only the circle 65 or only a portion thereof may be visualized. Needless to say that the image data 60 and any radius indication may need to be converted to a common scale for visualization.

Through the vertebra extends a spinal cord 61 that is not to be touched by the instrument tip 24. Since the visual representation 58 includes an indication of the radius 25, the user 18 can see when the instrument tip 24 comes too close to the spinal cord 61 is guided to adapt instrument movement accordingly. As the radius 25 has exactly been determined earlier, the corresponding visual guidance is very precise.

Optionally, the method may include generating display instructions for displaying a non-target region 63 in the image data 60 for which contact with the instrument 20 is to be avoided. In the example shown in Fig. 13A, the non-target region 63 is identical with the region of the spinal cord 61. Alternatively, the non-contact region may be larger than the spinal cord 61 or comprise a threshold region around the region of the spinal cord 61. The method may comprise tracking the position of the radius 25 of the instrument tip 24 and outputting a warning when the tracked instrument tip 24 comes in contact with the non-target region 63 and/or the threshold region. The warning may be at least one of an acoustic, optical, and haptic warning.

Fig. 13B shows a second example of a virtual space 47 with a visual representation 58 indicative of the radius 25 arranged relative to image data 60 of the vertebra (see Fig. 12). The visual representation 58 comprises the position of the centre point 27 and a radius indication 65 in form of a point arranged at a distance of the radius 25 from the centre point 27. The radius indication 65 covers less area of the image data 60 of the vertebra, giving the user 18 a better view of the image data 60. The radius indication 65 may be stationary (e.g., relative to an instrument axis 64 or the virtual space 47). Alternatively, the method may comprise determining a movement direction of the instrument tip 24 and generating display instructions for which the radius indication 65 moves towards the determined movement direction of the instrument tip 24. The radius indication 65 may be larger than a point.

As shown Fig. 13B, the visual representation 58 may further be indicative of the instrument axis 64. A pose of the instrument axis 64 may be determined on the basis of tracking the instrument tracker 22 while the surgical instrument 20 is rotated about the instrument axis 64. Alternatively or additionally, the instrument axis 64 may be determined based on tracking the instrument tracker 22 and having access to a known predetermined spatial relationship between the instrument tracker 22 and the instrument axis 64. The predetermined spatial relationship may be provided in form of a transformation that maps the pose of the instrument tracker 22 onto the pose of the instrument axis 64. The pose of the instrument axis 64 can therefore be determined by tracking the instrument tracker 22 in order to obtain the pose of the instrument tracker 22 and applying the transformation onto the pose of the instrument tracker 22.

Fig. 13C shows a third example of a virtual space 47 with a visual representation 58 of the instrument 20 arranged relative to image data 60 of an object in form of a bone surface. The visual representation 58 comprises the position of the centre point 27 (optional) and a sphere 66 around the centre point 27 with the determined radius 25. Displaying the radius 25 in form of a three dimensional sphere 66 may be used when the image data 60 or a tissue manipulation progress is also displayed in a three dimensional space. Fig. 13C shows a tissue manipulation progress 67 determined for a path of the visual representation 58. The tissue manipulation progress 67 may be determined based on the determined radius 25, a tracking of the instrument tracker 22, and the image data 60.

The method may further comprise outputting, on a display, the determined radius 25 of the spherically shaped surface portion 26 of the instrument tip 24 as a numerical value. The user 18 may be requested to confirm the numerical value prior to the surgical procedure.

The method may further comprise displaying, on a display (e.g., of the tracking system 14 or surgical navigation system 10), a visual representation of the instrument 20 arranged relative to the image data of the object. The visual representation of the instrument 20 may take the form of an icon.

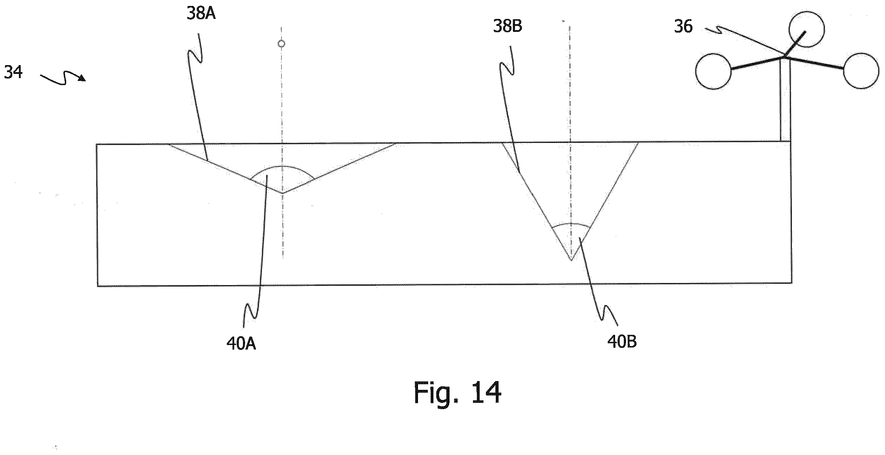

The calibration devices 34 described above only have a single calibration structure 38. However, a calibration device 34 may comprise a plurality of calibration structures 38. Such a calibration device 34 allows simultaneous calibration for a plurality of surgical instruments 20. Additionally, at least two of the calibration structures 38 may have a different opening angle 40. Fig. 14 shows a calibration device 34 with a first calibration structure 38A and a second calibration structure 38B. The first calibration structure 38A has a first opening angle 40A and the second calibration structure 38B has a second opening angle 40B, wherein the first opening angle 40A is larger than the second opening angle 40B. For the calibration structures 38A, 38B, the central axes are arranged in parallel. Alternatively, the central axes of the calibration structures 38A, 38B may be arranged in a non-parallel way (e.g., 45°, 90° or 135°).

The first opening angle 40A results in a larger diameter of the opening of the first calibration structure 38A and consequently allows insertion of an instrument tip 24 with a larger radius 25 compared to the second calibration structure 38B. On the other hand, the comparably smaller second opening angle 40b of the second calibration structure 38B translates a difference of the radius 25 of the instrument tip 24 to a larger difference of the apex distance 56 between the position of the apex 44 and the position of the centre point 27. Since the larger difference of the apex distance 56 can be better resolved with a limited spatial accuracy of the surgical navigation system 10, the second calibration structure 38B provides a more accurate radius determination. The calibration device 34 can therefore be used in combination with instrument tips 24 that have a much larger or smaller radius. As a result, a calibration device 34 with different opening angles 40A, 40B increases a range of usable tip radii 25 and consequently increases the flexibility of instrument selection for the user 18.

However, since the opening angle 40A, 40B is required for determining the radius 25, as an intermediate step, the method may include a step for determining the position and, optionally in case the opening angles 40A, 40B differ, the opening angle 40A, 40B of the calibration structure 38A, 38B in which the instrument tip is arranged by identifying the calibration structure 38A, 38B based on the position of the centre point 27.

The calibration structure 38A, 38B may be identified based the position of the centre point 27 and on the predetermined first relationship alone, e.g., based on a vicinity of the centre point 27 relative to the apex 44. Additional information may be provided, such as coordinates of a volume inside the calibration structure 38A, 38B. The calibration structure 38A, 38B may then be identified based on whether or not the position of the centre point 27 is inside the volume of either one of the calibration structures 38A, 38B.

In case a calibration structure 38A, 38B cannot be identified (e.g., the distance to the apex 44 exceeds a predetermined threshold, or the position of the centre point 27 is outside a volume of both calibration structures 38A, 38B), the method may comprise outputting an error message to the user 18.

Visualizing the radius 25 of the spherically shaped surface portion 26 gives access to valuable information for the user 18. The user 18 can obtain or verify the radius 25 right before moving the surgical instrument 20 in a surgical procedure, eliminating the risk of a mix up of an instrument tip 24 with a different radius and eliminating the need of managing labels or interaction with a user interface. The user 18 can consequently work more time efficiently and with a lower probability of causing harm to the patient. The surgical navigation system 10 gains the ability to improve accuracy of tracking the instrument tip 24, guiding the user 18, and planning tissue manipulation carried out with the instrument tip 24. The surgical procedure can therefore be carried out more efficiently and safely.

The tracking system 14 need not be attached to a surgical object such as the surgical instrument 20, the calibration device 34, or the patient. Instead, the tracking system 14 may be provided spatially separate from the surgical objects, overlooking the entire surgical environment 16. Such a position enables the tracking system 14 to track a plurality of surgical objects inside the surgical environment 16 and not only the surgical object to which the tracking system 14 is attached. When tracking a plurality of surgical objects, the surgical navigation system 10 is able to apply the determined radius 25 to interactions between the surgical instrument 20 and other tracked surgical objects. The surgical navigation system 10 tracks the surgical instrument 20 and the patient. Once the radius 25 has been determined, the device 12 can generate display instructions for visualizing the radius relative to the tissue of the patient. The accuracy of the surgical procedure is therefore improved.

The features described in relation to the exemplary embodiments shown in the drawings can be readily combined to result in different embodiments. It is apparent, therefore, that the present disclosure may be varied in many ways. Such variations are not to be regarded as a departure from the scope of the invention as defined by the claims appended hereto.

## Claims

1. A method (100) for enabling visualization of an indication of a radius (25) of a spherically shaped surface portion (26) of a tip (24) of an instrument (20) relative to image data (60) of an object (43) in a virtual space (47), wherein the spherically shaped surface portion (26) has a centre point (27), wherein a calibration device (34) is provided that comprises a calibration structure (38) defining an opening angle (40) and that is configured to cooperate with the spherically shaped surface portion (26) so as to guide a tilting movement of the instrument tip (24) around the centre point (27), wherein the calibration device (34) comprises a first tracker (36) trackable by a tracking system (14) and arranged in a predetermined relationship relative to the calibration structure (38), and wherein the instrument (20) comprises a second tracker (22) trackable by the tracking system (14) and the object (43) comprises a third tracker (41) trackable by the tracking system (14), the method (100) being performed by a computer system (13) and comprising:
determining (102) a first pose of the first tracker (36);
determining (104) positional data of the second tracker (22) for different tilting positions of the instrument (20) while the spherically shaped surface portion (26) is in abutment with the calibration structure (38);
determining (106) a position of the centre point (27) relative to the second tracker (22) based on the positional data and determining (106) the radius (25) of the spherically shaped surface portion (26) based on the first pose, the positional data, the predetermined relationship, and the opening angle (40);
tracking (108) the third tracker (41) to track a second pose of the image data (60) of the object (43) in the virtual space (47);
tracking (110) the second tracker (22) to track a position of the centre point (27) in the virtual space (47) based on the position of the centre point (27) relative to the second tracker (22); and
generating (112) first display instructions for displaying in the virtual space (47), based on the second pose of the image data (60) of the object (43) in the virtual space (47), the position of the centre point (27) in the virtual space (47) and the radius (25), a visual representation (58) indicative of the radius (25) relative to the image data (60) of the object (43).

2. The method (100) according to claim 1, comprising
determining the radius (25) by multiplying the sinus of half of the opening angle (40) with a distance (56) between the position of the centre point (27) and a real or imaginary point (44) relative to which the opening angle (40) is defined.

3. The method (100) according to claim 2, wherein
the distance (56) between the position of the centre point (27) and the real or imaginary point (44) is determined based on the first pose, the predetermined relationship, the positional data and the position of the centre point (27).

4. The method (100) according to any of the preceding claims, wherein
the positional data comprises at least two different third poses of the second tracker (22) for the different tilting positions.

5. The method (100) according to any of the preceding claims, wherein
the positional data comprises at least four different positions of the second tracker (22) for the different tilting positions.

6. The method (100) according to any of the preceding claims, comprising
outputting, on a display, user instructions to tilt the instrument (20) for calibration purposes.

7. The method (100) according to any of the preceding claims, further comprising
generating second display instructions for displaying a non-target region (63) in the image data (60) for which contact with the instrument (20) is to be avoided.

8. The method (100) according to any of the preceding claims, comprising
displaying, on a display, a visual representation (58) of at least a portion of the instrument (20) relative to the image data (60) of the object (43).

9. The method (100) according to any of the preceding claims, wherein
the calibration structure (38) has a shape of a cone or a pyramid, or portion thereof, defining the opening angle (40).

10. The method (100) according to any of the preceding claims, wherein
the visual representation (58) comprises an icon representative of at least a part of a circle (65) or a sphere (66) with the radius (25) of the spherically shaped surface portion (26).

11. The method (100) according to any of the preceding claims, wherein
a pose of an instrument axis (64) is determined on the basis of one of
- tracking the second tracker (22) while the instrument (20) is rotated about the instrument axis (64), or
- tracking the second tracker (22) and evaluating a known predetermined spatial relationship between the second tracker (22) and the instrument axis (64),
wherein the visual representation (58) is indicative of the pose of the instrument axis (64) in the virtual space (47).

12. A computer program product, comprising instructions that, when executed on at least one processor, cause the at least one processor to carry out any the methods of claims 1 to 11.

13. A device (12) for enabling visualization of an indication of a radius (25) of a spherically shaped surface portion (26) of a tip (24) of an instrument (20) relative to image data (60) of an object (43) in a virtual space (47), wherein the spherically shaped surface portion (26) has a centre point (27) and is configured to cooperate with a calibration structure (38) defining an opening angle (40) of a calibration device (34) so that a tilting movement of the instrument tip (24) is guided around the centre point (27), wherein the calibration device (34) comprises a first tracker (36) trackable by a tracking system (14) and arranged in a predetermined relationship relative to the calibration structure (38), and wherein the instrument (20) comprises a second tracker (22) trackable by the tracking system (14) and the object (43) comprises a third tracker (41) trackable by the tracking system (14), the device (12) being configured to:
determine a first pose of the first tracker (36);
determine positional data of the second tracker (22) for different tilting positions of the instrument (20) while the spherically shaped surface portion (26) is in abutment with the calibration structure (38);
determine a position of the centre point (27) relative to the second tracker (22) based on the positional data and determine the radius (25) of the spherically shaped surface portion (26) based on the first pose, the positional data, the predetermined relationship, and the opening angle (40);
track the third tracker (41) to track a second pose of the image data (60) of the object (43) in the virtual space (47);
track the second tracker (22) to track a position of the centre point (27) in the virtual space (47) based on the position of the centre point (27) relative to the second tracker (22); and
generate first display instructions for displaying in the virtual space (47), based on the second pose of the image data (60) of the object (43) in the virtual space (47), the position of the centre point (27) in the virtual space (47) and the radius (25), a visual representation (58) indicative of the radius (25) relative to the image data (60) of the object (43).

14. The device (12) according to claim 13, configured to perform any of the method steps according to claims 2 to 11.

15. A calibration system (15) comprising
the device (12) according to claim 13 or 14;
the calibration device (34) with the first tracker (36) coupled thereto; and
the instrument (20) with the second tracker (22) coupled thereto.

16. A surgical navigation system (10) comprising
the calibration system (15) according to claim 15;
the tracking system (14), wherein the tracking system (14) comprises or is communicatively coupled with the device (12) according to claim 13 or 14.

## Patentansprüche

1. Verfahren (100) zum Ermöglichen der Visualisierung einer Anzeige eines Radius (25) eines sphärisch geformten Oberflächenabschnitts (26) einer Spitze (24) eines Instruments (20) relativ zu Bilddaten (60) eines Objekts (43) in einem virtuellen Raum (47), wobei der sphärisch geformte Oberflächenabschnitt (26) einen Mittelpunkt (27) aufweist, wobei eine Kalibrierungsvorrichtung (34) vorgesehen ist, die eine Kalibrierungsstruktur (38) umfasst, die einen Öffnungswinkel (40) definiert und die so konfiguriert ist, dass sie mit dem sphärisch geformten Oberflächenabschnitt (26) zusammenwirkt, um eine Neigungsbewegung der Instrumentenspitze (24) um den Mittelpunkt (27) zu führen, wobei die Kalibrierungsvorrichtung (34) einen ersten Tracker (36) umfasst, der durch ein Verfolgungssystem (14) verfolgbar ist und in einer vorbestimmten Beziehung relativ zu der Kalibrierungsstruktur (38) angeordnet ist, und wobei das Instrument (20) einen zweiten Tracker (22) umfasst, der durch das Verfolgungssystem (14) verfolgbar ist, und das Objekt (43) einen dritten Tracker (41) umfasst, der durch das Verfolgungssystem (14) verfolgbar ist, wobei das Verfahren (100) durch ein Computersystem (13) durchgeführt wird und umfasst:
Bestimmen (102) einer ersten Pose des ersten Trackers (36);
Bestimmen (104) von Positionsdaten des zweiten Trackers (22) für verschiedene Neigungspositionen des Instruments (20), während der sphärisch geformte Oberflächenabschnitt (26) an der Kalibrierungsstruktur (38) anliegt;
Bestimmen (106) einer Position des Mittelpunkts (27) relativ zu dem zweiten Tracker (22) basierend auf den Positionsdaten, und Bestimmen (106) des Radius (25) des sphärisch geformten Oberflächenabschnitts (26) basierend auf der ersten Pose, den Positionsdaten, der vorbestimmten Beziehung und dem Öffnungswinkel (40);
Verfolgen (108) des dritten Trackers (41), um eine zweite Pose der Bilddaten (60) des Objekts (43) im virtuellen Raum (47) zu verfolgen;
Verfolgen (110) des zweiten Trackers (22), um eine Position des Mittelpunkts (27) im virtuellen Raum (47) basierend auf der Position des Mittelpunkts (27) relativ zu dem zweiten Tracker (22) zu verfolgen; und
Erzeugen (112) erster Anzeigeanweisungen, um basierend auf der zweiten Pose der Bilddaten (60) des Objekts (43) in dem virtuellen Raum (47), der Position des Mittelpunkts (27) in dem virtuellen Raum (47) und dem Radius (25) eine visuelle Darstellung (58) in dem virtuellen Raum (47), die den Radius (25) relativ zu den Bilddaten (60) des Objekts (43) anzeigt, anzuzeigen.

2. Verfahren (100) nach Anspruch 1, umfassend
Bestimmen des Radius (25) durch Multiplikation des Sinus der Hälfte des Öffnungswinkels (40) mit einem Abstand (56) zwischen der Position des Mittelpunkts (27) und einem realen oder imaginären Punkt (44), relativ zu dem der Öffnungswinkel (40) definiert ist.

3. Verfahren (100) nach Anspruch 2, wobei
der Abstand (56) zwischen der Position des Mittelpunkts (27) und dem realen oder imaginären Punkt (44) basierend auf der ersten Pose, der vorbestimmten Beziehung, den Positionsdaten und der Position des Mittelpunkts (27) bestimmt wird.

4. Verfahren (100) nach einem der vorhergehenden Ansprüche, wobei
die Positionsdaten mindestens zwei verschiedene dritte Posen des zweiten Trackers (22) für die verschiedenen Neigungspositionen umfassen.

5. Verfahren (100) nach einem der vorhergehenden Ansprüche, wobei
die Positionsdaten mindestens vier verschiedene Positionen des zweiten Trackers (22) für die verschiedenen Neigungspositionen umfassen.

6. Verfahren (100) nach einem der vorhergehenden Ansprüche, umfassend
Ausgeben von Benutzeranweisungen zum Neigen des Instruments (20) zu Kalibrierungszwecken auf einer Anzeige.

7. Verfahren (100) nach einem der vorhergehenden Ansprüche, ferner umfassend
Erzeugen zweiter Anzeigeanweisungen, um einen Nicht-Zielbereich (63) in den Bilddaten (60), für den ein Kontakt mit dem Instrument (20) vermieden werden soll, anzuzeigen.

8. Verfahren (100) nach einem der vorhergehenden Ansprüche, umfassend
Anzeigen einer visuellen Darstellung (58) von mindestens einem Abschnitt des Instruments (20) relativ zu den Bilddaten (60) des Objekts (43) auf einer Anzeige.

9. Verfahren (100) nach einem der vorhergehenden Ansprüche, wobei
die Kalibrierungsstruktur (38) die Form eines Kegels oder einer Pyramide oder eines Abschnitts davon hat, der/die den Öffnungswinkel (40) definiert.

10. Verfahren (100) nach einem der vorhergehenden Ansprüche, wobei
die visuelle Darstellung (58) ein Symbol umfasst, das zumindest einen Teil eines Kreises (65) oder einer Kugel (66) mit dem Radius (25) des sphärisch geformten Oberflächenabschnitts (26) darstellt.

11. Verfahren (100) nach einem der vorhergehenden Ansprüche, wobei
eine Pose einer Instrumentenachse (64) bestimmt wird auf der Basis von einem von
- Verfolgen des zweiten Trackers (22), während das Instrument (20) um die Instrumentenachse (64) rotiert wird, oder
- Verfolgen des zweiten Trackers (22) und Auswerten einer bekannten vorgegebenen räumlichen Beziehung zwischen dem zweiten Tracker (22) und der Instrumentenachse (64),
wobei die visuelle Darstellung (58) die Pose der Instrumentenachse (64) im virtuellen Raum (47) anzeigt.

12. Computerprogrammprodukt, umfassend Anweisungen, die, wenn sie auf mindestens einem Prozessor ausgeführt werden, den mindestens einen Prozessor dazu veranlassen, eines der Verfahren der Ansprüche 1 bis 11 durchzuführen.

13. Vorrichtung (12) zum Ermöglichen der Visualisierung einer Anzeige eines Radius (25) eines sphärisch geformten Oberflächenabschnitts (26) einer Spitze (24) eines Instruments (20) relativ zu Bilddaten (60) eines Objekts (43) in einem virtuellen Raum (47), wobei der sphärisch geformte Oberflächenabschnitt (26) einen Mittelpunkt (27) aufweist und so konfiguriert ist, dass er mit einer Kalibrierungsstruktur (38) zusammenwirkt, die einen Öffnungswinkel (40) einer Kalibrierungsvorrichtung (34) definiert, so dass eine Neigungsbewegung der Instrumentenspitze (24) um den Mittelpunkt (27) geführt wird, wobei die Kalibrierungsvorrichtung (34) einen ersten Tracker (36) umfasst, der durch ein Verfolgungssystem (14) verfolgbar ist und in einer vorbestimmten Beziehung relativ zu der Kalibrierungsstruktur (38) angeordnet ist, und wobei das Instrument (20) einen zweiten Tracker (22) umfasst, der durch das Verfolgungssystem (14) verfolgbar ist, und das Objekt (43) einen dritten Tracker (41) umfasst, der durch das Verfolgungssystem (14) verfolgbar ist, wobei die Vorrichtung (12) konfiguriert ist, um:
eine erste Pose des ersten Trackers (36) zu bestimmen;
Positionsdaten des zweiten Trackers (22) für verschiedene Neigungspositionen des Instruments (20) zu bestimmen, während der sphärisch geformte Oberflächenabschnitt (26) an der Kalibrierstruktur (38) anliegt;
eine Position des Mittelpunkts (27) relativ zu dem zweiten Tracker (22) basierend auf den Positionsdaten zu bestimmen, und den Radius (25) des sphärisch geformten Oberflächenabschnitts (26) basierend auf der ersten Pose, den Positionsdaten, der vorbestimmten Beziehung und dem Öffnungswinkel (40) zu bestimmen;
den dritten Tracker (41) zu verfolgen, um eine zweite Pose der Bilddaten (60) des Objekts (43) im virtuellen Raum (47) zu verfolgen;
den zweiten Tracker (22) zu verfolgen, um eine Position des Mittelpunkts (27) in dem virtuellen Raum (47) basierend auf der Position des Mittelpunkts (27) relativ zu dem zweiten Tracker (22) zu verfolgen; und
erste Anzeigeanweisungen zu erzeugen, um basierend auf der zweiten Pose der Bilddaten (60) des Objekts (43) im virtuellen Raum (47), der Position des Mittelpunkts (27) im virtuellen Raum (47) und dem Radius (25) eine visuelle Darstellung (58) im virtuellen Raum (47) anzuzeigen, die den Radius (25) relativ zu den Bilddaten (60) des Objekts (43) anzeigt.

14. Vorrichtung (12) nach Anspruch 13, die konfiguriert ist, um einen der Verfahrensschritte nach einem der Ansprüche 2 bis 11 durchzuführen.

15. Kalibrierungssystem (15), umfassend
die Vorrichtung (12) nach Anspruch 13 oder 14;
die Kalibrierungsvorrichtung (34) mit dem daran gekoppelten ersten Tracker (36); und
das Instrument (20) mit dem daran gekoppelten zweiten Tracker (22).

16. Chirurgisches Navigationssystem (10), umfassend
das Kalibrierungssystem (15) nach Anspruch 15;
das Verfolgungssystem (14), wobei das Verfolgungssystem (14) die Vorrichtung (12) nach Anspruch 13 oder 14 umfasst oder mit dieser kommunikativ gekoppelt ist.

## Revendications

1. Procédé (100) pour permettre la visualisation d'une indication d'un rayon (25) d'une partie (26) de surface sphérique d'un embout (24) d'un instrument (20) par rapport à des données d'image (60) d'un objet (43) dans un espace virtuel (47), dans lequel la partie (26) de surface sphérique a un point central (27), dans lequel un dispositif d'étalonnage (34) comprend une structure d'étalonnage (38) définissant un angle d'ouverture (40) et est conçu pour coopérer avec la partie (26) de surface sphérique de manière à guider un mouvement d'inclinaison de l'embout de l'instrument (24) autour du point central (27), dans lequel le dispositif d'étalonnage (34) comprend un premier suiveur (36) pouvant être suivi par un système de suivi (14) et se trouvant dans une relation prédéterminée par rapport à la structure d'étalonnage (38), et dans lequel l'instrument (20) comprend un deuxième suiveur (22) pouvant être suivi par le système de suivi (14) et l'objet (43) comprend un troisième suiveur (41) pouvant être suivi par le système de suivi (14), le procédé (100) étant mis en œuvre par un système informatique (13) et comprenant:
la détermination (102) d'une première position du premier suiveur (36);
la détermination (104) de données de position du deuxième suiveur (22) pour différentes positions d'inclinaison de l'instrument (20) lorsque la partie (2-) de surface sphérique est en butée avec la structure d'étalonnage (38);
la détermination (106) d'une position du point central (27) par rapport au deuxième suiveur (22) sur la base des données de position et la détermination (106) du rayon (25) de la partie (26) de surface sphérique en fonction de la première pose, des données de position, de la relation prédéterminée et de l'angle d'ouverture (40);
le suivi (108) du troisième suiveur (41) pour suivre une deuxième pose des données d'image (60) de l'objet (43) dans l'espace virtuel (47);
le suivi (110) du deuxième suiveur (22) pour suivre une position du point central (27) dans l'espace virtuel (47) sur la base de la position du point central (27) par rapport au deuxième suiveur (22); et
la génération (112) de premières instructions d'affichage pour afficher dans l'espace virtuel (47), sur la base de la deuxième pose de l'objet des données d'image (60) de l'objet (43) dans l'espace virtuel (47), de la position du point central (27) dans l'espace virtuel (47) et du rayon (25), une représentation visuelle (58) indiquant le rayon (25) par rapport aux données d'image (60) de l'objet (43).

2. Procédé (100) selon la revendication 1, comprenant
la détermination du rayon (25) par multiplication du sinus de la moitié de l'angle d'ouverture (40) par une distance (56) entre la position du point central (27) et un point réel ou imaginaire (44) par rapport auquel est défini l'angle d'ouverture (40).

3. Procédé (100) selon la revendication 2, dans lequel
la distance (56) entre la position du point central (27) et le point réel ou imaginaire (44) est déterminée sur la base de la première pose, de la relation prédéterminée, des données de position et de la position du point central (27).

4. Procédé (100) selon l'une quelconque des revendications précédentes, dans lequel
les données de position comprennent au moins deux troisièmes positions différentes du deuxième suiveur (22) pour les différentes positions d'inclinaison.

5. Procédé (100) selon l'une quelconque des revendications précédentes, dans lequel
les données de position comprennent au moins quatre positions différentes du deuxième suiveur (22) pour les différentes positions d'inclinaison.

6. Procédé (100) selon l'une quelconque des revendications précédentes, comprenant
l'affichage, sur un écran, d'instructions d'utilisateur permettant d'incliner l'instrument (20) à des fins d'étalonnage.

7. Procédé (100) selon l'une quelconque des revendications précédentes, comprenant en outre
la génération de secondes instructions d'affichage permettant d'afficher une région non cible (63) dans les données d'image (60), laquelle région ne doit pas entrer en contact l'instrument (20).

8. Procédé (100) selon l'une quelconque des revendications précédentes, comprenant
l'affichage, sur un écran, d'une représentation visuelle (58) d'au moins une partie de l'instrument (20) par rapport aux données d'image (60) de l'objet (43).

9. Procédé (100) selon l'une quelconque des revendications précédentes, dans lequel
la structure d'étalonnage (38) a la forme d'un cône ou d'une pyramide, ou une partie de ces derniers, définissant l'angle d'ouverture (40).

10. Procédé (100) selon l'une quelconque des revendications précédentes, dans lequel
la représentation visuelle (58) comprend une icône représentant au moins une partie d'un cercle (65) ou d'une sphère (66) avec le rayon (25) de la partie (26) de surface sphérique.

11. Procédé (100) selon l'une quelconque des revendications précédentes, dans lequel
la position d'un axe d'instrument (64) est déterminée sur la base de l'une des actions suivantes
- suivre le deuxième suiveur (22) pendant que l'instrument (20) tourne autour de l'axe de l'instrument (64), ou
- suivre le deuxième suiveur (22) et évaluer une relation spatiale prédéterminée connue entre le deuxième suiveur (22) et l'axe de l'instrument (64),
dans lequel la représentation visuelle (58) indique la position de l'axe de l'instrument (64) dans l'espace virtuel (47).

12. Produit programme informatique, comprenant des instructions qui, lorsqu'elles sont exécutées sur au moins un processeur, amènent l'au moins un processeur à mettre en œuvre l'un des procédés selon les revendications 1 à 11.

13. Dispositif (12) pour permettre la visualisation d'une indication d'un rayon (25) d'une partie (26) de surface sphérique d'un embout (24) d'un instrument (20) par rapport à des données d'image (60) d'un objet (43) dans un espace virtuel (47), dans lequel la partie (26) de surface sphérique a un point central (27) et est conçu pour coopérer avec une structure d'étalonnage (38) définissant un angle d'ouverture (40) du dispositif d'étalonnage (34) de sorte qu'un mouvement d'inclinaison de l'embout d'instrument (24) est guidé autour du point central (27), dans lequel le dispositif d'étalonnage (34) comprend un premier suiveur (36) pouvant être suivi par un système de suivi (14) et se trouvant dans une relation prédéterminée par rapport à la structure d'étalonnage (38), et dans lequel l'instrument (20) comprend un deuxième suiveur (22) pouvant être suivi par le système de suivi (14) et l'objet (43) comprend un troisième suiveur (41) pouvant être suivi par le système de suivi (14), le dispositif (12) étant conçu pour:
déterminer une première pose du premier suiveur (36);
déterminer des données de position du deuxième suiveur (22) pour différentes positions d'inclinaison de l'instrument (20) lorsque la partie de surface de forme sphérique (26) est en butée avec la structure d'étalonnage (38);
déterminer une position du point central (27) par rapport au deuxième suiveur (22) sur la base des données de position et déterminer le rayon (25) de la partie (26) de surface sphérique en fonction de la première pose, des données de position, de la relation prédéterminée et de l'angle d'ouverture (40);
suivre (108) le troisième suiveur (41) pour suivre une deuxième pose des données d'image (60) de l'objet (43) dans l'espace virtuel (47);
suivre (110) le deuxième suiveur (22) pour suivre une position du point central (27) dans l'espace virtuel (47) sur la base de la position du point central (27) par rapport au deuxième suiveur (22); et
générer des premières instructions d'affichage pour afficher dans l'espace virtuel (47), sur la base de la seconde pose de l'objet des données d'image (60) de l'objet (43) dans l'espace virtuel (47), de la position du point central (27) dans l'espace virtuel (47) et du rayon (25), une représentation visuelle (58) indiquant le rayon (25) par rapport aux données d'image (60) de l'objet (43).

14. Dispositif (12) selon la revendication 13, configuré pour effectuer l'une quelconque des étapes du procédé selon les revendications 2 à 11.

15. Système d'étalonnage (15) comprenant
le dispositif (12) selon la revendication 13 ou 14;
le dispositif d'étalonnage (34) auquel est accouplé le premier suiveur (36); et l'instrument (20) auquel est accouplé le deuxième suiveur (22).

16. Système de navigation chirurgicale (10) comprenant
le système d'étalonnage (15) selon la revendication 15;
le système de suivi (14), le système de suivi (14) comprenant ou étant en communication avec le dispositif (12) selon la revendication 13 ou 14.
